# EUROPEAN PATENT APPLICATION

(11) **EP 1 174 094 A1**
(43) Date of publication of application: **23.01.2002**
(21) Application number: 00202323.2
(22) Date of filing: 03.07.2000
(51) Int. Cl.: A61C 8/00

(54) **Constructive disposition introduced in an auxiliary device applied in dental surgeries for implants**

(71) Applicant: Yuiti Katayama, Americo, Sao Paulo, SP (BR); Nicolau Rodrigues, Geraldo, Sao Paulo, SP (BR)
(72) Inventor: Yuiti Katayama, Americo, Sao Paulo, SP (BR); Nicolau Rodrigues, Geraldo, Sao Paulo, SP (BR)
(74) Representative: Elzaburu Marquez, Alberto

(57) **Abstract**

Constructive disposition introduced in an auxiliary device applied in dental surgeries for implants, wherein the auxiliary device basically consists of a mini-balloon (4) made of P.V.C., latex or silicone, which is connected to the narrow end of a P.V.C. hose (3) while the other end is connected to an ordinary syringe (2); in this way, to the realization of the access surgery to the Schenider membrane (m) and the placement of the implant or in some cases only graft, a small drilling (4 millimeters) is executed in the alveolar bone, through which the deflated mini-balloon is introduced. Once installed, the balloon is inflated with some cubic centimeters of a sterile physiologic saline solution, air or sterile water, displacing the membrane and making possible the previous knowledge of the graft bone amount that will be necessary; so, it is not necessary anymore to make a side window in the maxillary bone, neither it is necessary anymore to use metallic curets for displacement of the membrane; thus, the surgery is made in a faster and less traumatic way, and the recovering time of the operated area is reduced.

## Description

The present invention relates to a new disposition introduced in an auxiliary device applied in dental surgeries for implants, more specifically to a new mechanical device that facilitates the procedure of the lift of the "Schenider membrane" causing no harm or greater injuries to it, providing a shorter recovery time to the operated area.

Some surgeries are needed when the bone available is not enough to place the implants in the maxillary posterior quadrant, being necessary to make the augmentation of the maxillary sinus floor, referred to as a *sinus floor augmentation.*

As it is well known by the experts in this area, and as an illustration, this surgery is made by using surgical motor and burs, being very long and extremely delicate.

First of all a "window" is opened in the bone through the burs and using metallic curets the displacement of the Schenider membrane is carried out, everything possible being made to avoid the rupture of the membrane, because if it happens the implant surgery will not be performed due to the contamination by the air that comes from the nose; as a simple comparison this surgery is the same as "cutting an egg shell without harming its internal membrane".

Before these difficulties and with the purpose to eliminate them it was developed by the Applicants the present constructive disposition in an auxiliary device applied in dental surgeries for implants, which makes possible to the patient a reduced trauma and a better recovery, while offering to the surgeon a better control and confidence that the Schenider membrane will not tear up.

This auxiliary device is basically composed of a mini-balloon made of P.V.C., latex or even silicone, which connects to the end of a narrow P.V.C. hose while the other end is adapted to an ordinary syringe.

In this way, in order to perform the access to the Schenider membrane and to place the implants, a small drilling (4 millimeters) is made in the alveolar bone, through which the deflated mini-balloon is introduced. Once placed, the mini-balloon is inflated with some cm³ of air, sterile physiologic saline solution or sterile water, displacing the membrane, making possible the previous knowledge of the bone graft amount that will be necessary.

Thus, through this method it is not necessary to make a side window in the maxillary bone, neither it is necessary to utilize metallic curets to displace the membrane, and so the surgery is performed in a faster and less traumatic way and the recovery time is extremely short.

The present invention will be described in full details according to the annexed drawings in which:

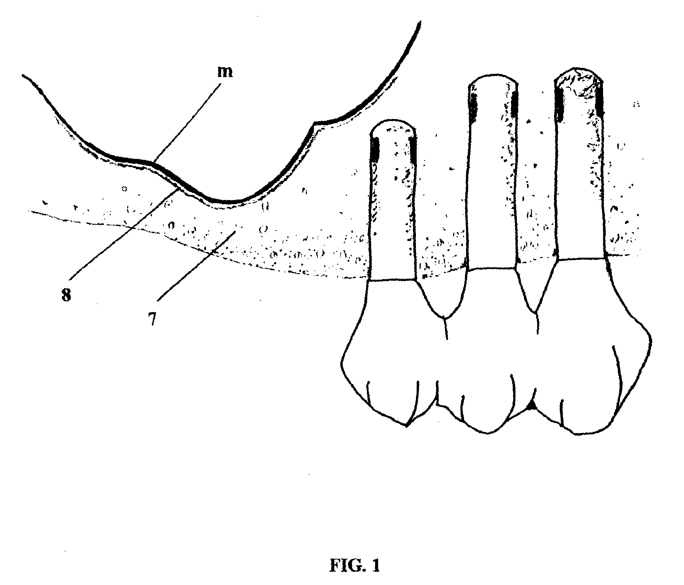

Figure 1 is a schematic view of the area (7) for the lifting of the membrane (m);

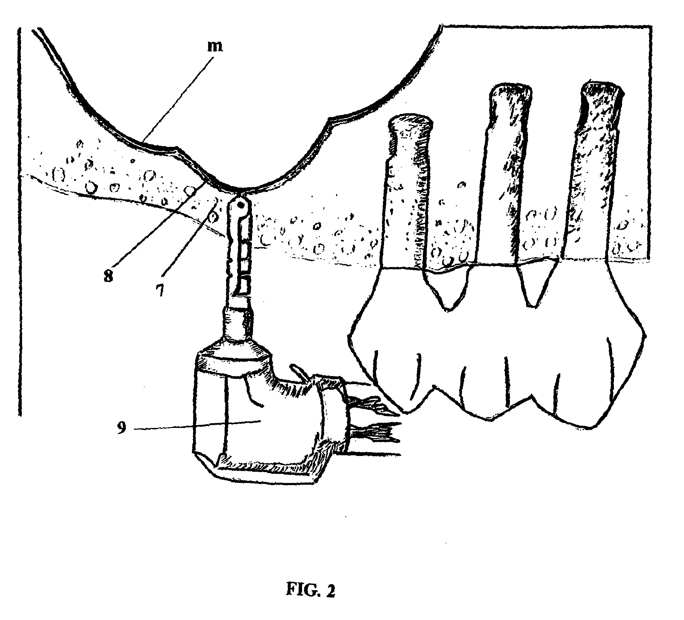

Figure 2 is a schematic view with the access to the cortical bone (8) by means of a bur (9);

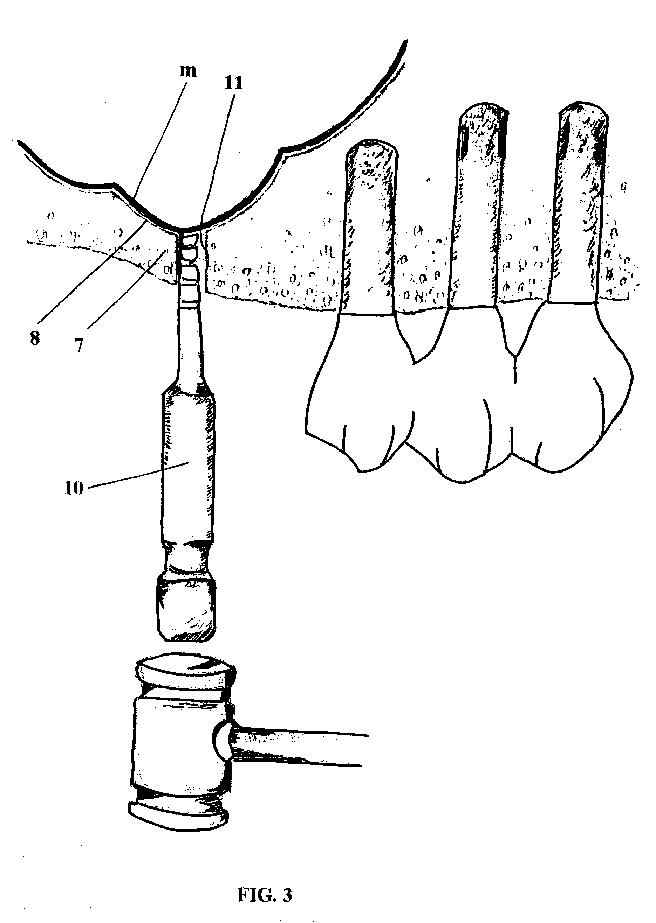

Figure 3 is a schematic view with the access to the membrane (m) through the osteotome (10) ;
Figure 4 shows the S.L.B. (Sinus Lifting Balloon) deflated coupled to a syringe. The balloon deflated has 2.8 millimeters of diameter. We can predetermine the expansion volume according to the amount of air or physiologic saline solution present in a syringe and at same time we can determinate the amount of bone graft necessary;
Figure 5 shows the S.L.B. in action. We can see the balloon expansion according to the amount of air or physiologic saline solution in cubic centimeters injected into the balloon;

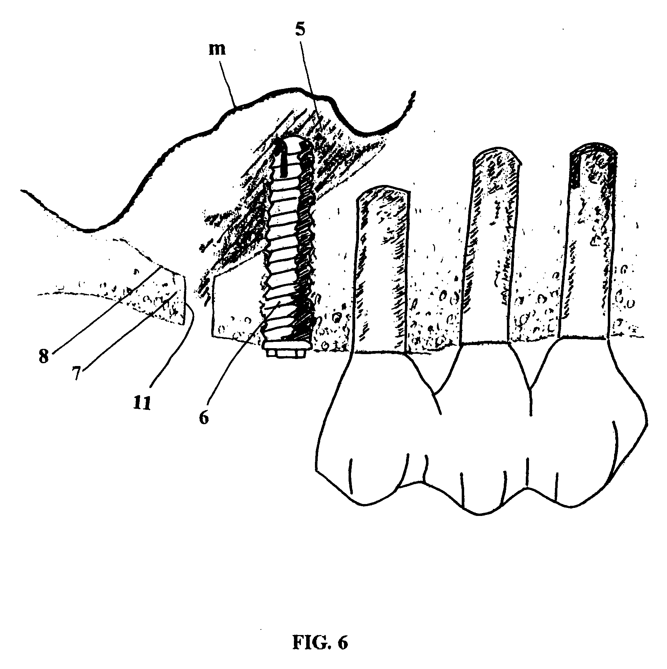

Figure 6 is a schematic view after the membrane lifting with the implant installation and lifting of the sinus with bone graft; and

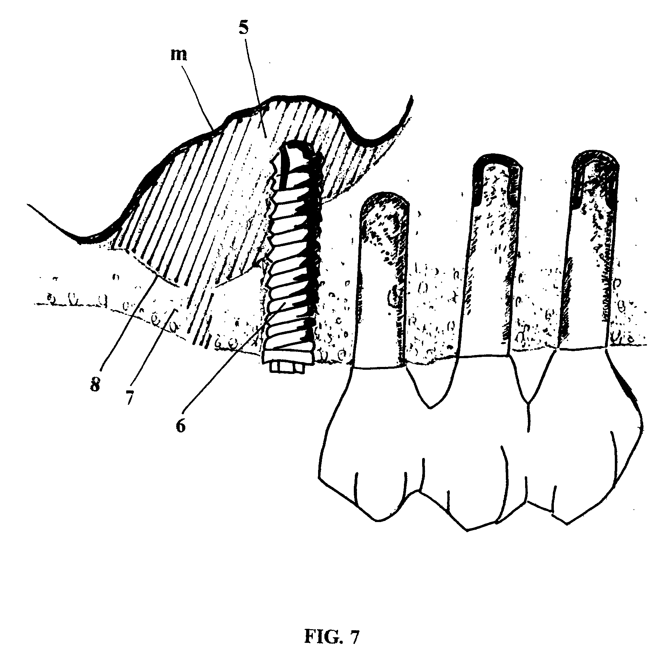

Figure 7 is a schematic view of the bone graft in the maxillary sinus.

According to the figures previously described, the new constructive disposition introduced in an auxiliary device applied in dental surgeries for implants, subject of present invention, is obtained through the device (1), formed by the syringe (2), which has connected, in its injector point (2a), one end of a P.V.C. hose (3), which has the other end connected to a mini-balloon (4) inserted through a small drilling (11) in the bone area (7).

The referred device has its operation based on the injection of some cm³ of the sterile physiologic saline solution contained in the syringe (2) into the interior of the mini-balloon (4), being so that the cubic metric volume of the injected component has visual correspondence obtained through the scale (E) applied to the body of the syringe.

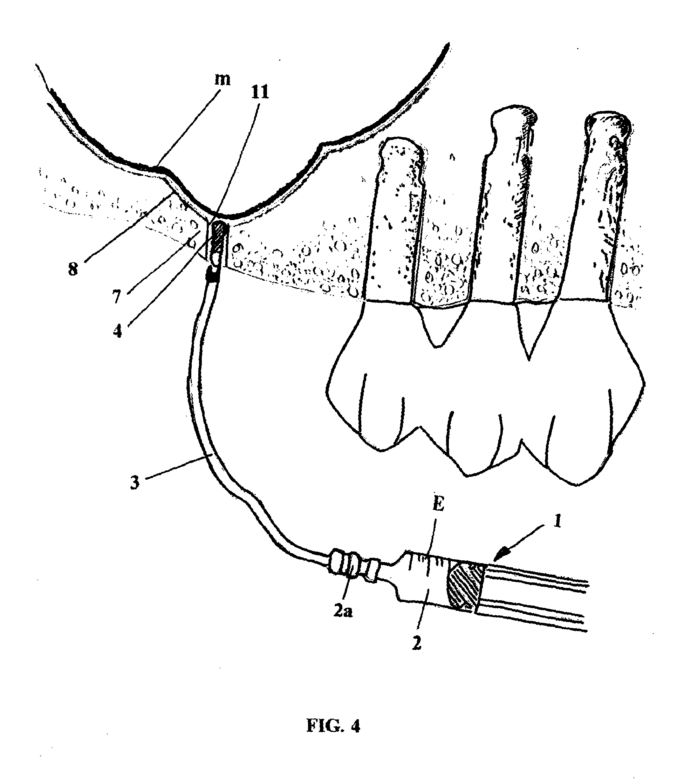

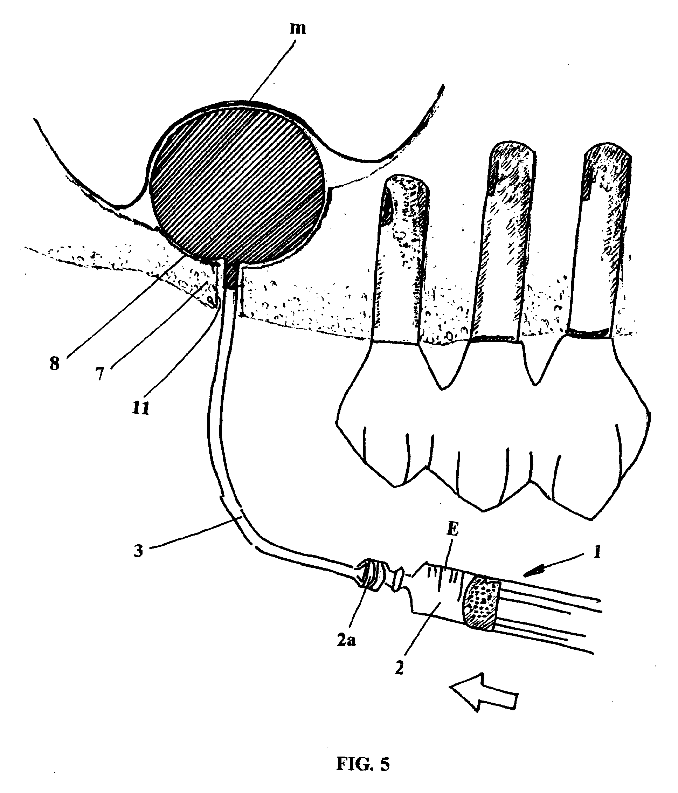

The application method of the device (1) that we are talking about (see figures 4 and 5) is made of: a) localization of the graft and implant sites; b) reduced surgical opening in the bone (4 millimeters); c) introduction of the mini-balloon (4) in deflated condition; d) injection of the sterile physiologic saline solution into the mini-balloon (4) through the syringe (2), with the soft displacement of the membrane (m) without rupture risk; d1) new injection of the sterile physiologic saline solution to a broader displacement; e) once the membrane is displaced, a cavity for the bone graft (5) is obtained, being of the adequate quality for the bone graft (5) that was previously stipulated by visual measuring of the scale (E) in cubic centimeters of sterile physiologic saline solution injected into the mini-balloon (4); f) introduction of the implant (6); and g) total closing of surgical area.

In this way the surgical access to the "Schenider membrane" becomes a lot easier to be carried out by the surgeon and with the recovering a lot less traumatic for the patient.

Although a preferred embodiment of the present invention has been described and illustrated in the foregoing, it must be understood that countless changings may be made to the presented description, and the protection goal is defined by the claimed subject that follows.

## Claims

1. Constructive disposition introduced in an auxiliary device applied in dental surgeries for implants, **characterized in that** the auxiliary device (1) is formed by a syringe (2) which has connected in its injector point (2a) the end of a P.V.C. hose (3) which has the other end connected to a mini-balloon (4), said device having its operation based on the injection of some cubic centimeters of sterile physiologic saline solution, sterile water or air contained in the syringe (2) into the interior of the mini-balloon (4), being so that the cubic metric volume of the injected component makes possible the previous knowledge of the graft bone amount that will be necessary, through the scale (E) applied to the syringe body.

2. Method of using the constructive disposition introduced in an auxiliary device applied in dental surgeries for implants according to claim 1, **characterized in that** the device (1) application method consists of: a) localization of the implant and graft area; b) reduced opening of the surgical access (4 millimeters) ; c) introduction of the mini-balloon (4) in deflated condition; d) injection of sterile physiologic saline solution, sterile water or air into the mini-balloon (4) through the syringe (2) with soft displacement of the membrane (m) without rupture risk; dl) new injection of the sterile physiologic saline solution, sterile water or air to a broader displacement; e) once the membrane is displaced, an area for the bone graft (5) is obtained, being of the adequate quality for the bone graft (5) that was previously stipulated by visual measuring of the scale (E) in cubic centimeters of sterile physiologic saline solution injected into the mini-balloon (4); f) introduction of the implant (6); and g) total closing of the surgical area.
